# EUROPEAN PATENT APPLICATION

(11) **EP 4 564 358 A1**
(43) Date of publication of application: **04.06.2025**
(21) Application number: 23846327.7
(22) Date of filing: 19.07.2023
(51) Int. Cl.: G16C 60/00

(54) **CHARACTERISTICS PREDICTION DEVICE, CHARACTERISTICS PREDICTION METHOD, AND PROGRAM**

(30) Priority: 27.07.2022 JP 2022119644
(71) Applicant: Resonac Corporation, Tokyo 105-7325 (JP)
(72) Inventor: LEE, Haein, Tokyo 105-8518 (JP); TAKEMOTO, Shimpei, Tokyo 105-8518 (JP); OKUNO, Yoshishige, Tokyo 105-8518 (JP)
(74) Representative: Strehl & Partner mbB
(86) International application number: PCT/JP2023/026453
(87) International publication number: WO 2024/024596

(57) **Abstract**

A relation between design conditions and property prediction values of a target substance is visualized to be easily grasped. A standard design determination unit configured to determine a standard design condition representing a design condition in a target substance produced using two or more material substances, a neighboring design generation unit configured to generate a plurality of neighboring design conditions by changing one design value with respect to the standard design condition, a property prediction unit configured to predict two or more property values under each of the plurality of neighboring design conditions, and a visualization unit configured to arrange symbols corresponding to the plurality of neighboring design conditions, on a plane having the design value and a first property value as axes, in a color representation corresponding to a second property value, are included.

## Description

### TECHNICAL FIELD

The present disclosure relates to a property prediction device, a property prediction method, and a program.

### BACKGROUND

Conventionally, material development has been performed based on experience, intuitions, and the like of engineers. In recent years, a technique for improving efficiency of material development by utilizing machine learning or the like is used.

For example, Patent Document 1 discloses an invention of predicting a physical property of a composition composed of multiple materials, and displaying a feature of a specific material contained in the composition and a prediction value of the physical property in association with each other.

### Related Art Document

### Patent Document

[Patent Document 1] Japanese Patent Application Laid-Open No. 2022-128962

### SUMMARY OF THE INVENTION

### Problem to be solved by the invention

However, in the related art, there is a problem in that it is difficult to grasp a change tendency in multiple features or multiple physical property values. For example, in the invention disclosed in Patent Document 1, in order to grasp a change tendency in multiple features or multiple physical property values, it is necessary to display and compare multiple coordinate systems representing a relationship between the feature and the physical property value.

In view of the technical problem as described above, it is an object of the present disclosure to visualize a relationship between a design value and a property value of a target substance so that the relationship can be easily grasped.

### Means for Solving Problem

The present disclosure includes the following configurations.
[1] A property prediction device including:
   a standard design determination unit configured to determine a standard design condition representing a design condition in a target substance produced using two or more material substances;
   a neighboring design generation unit configured to generate a plurality of neighboring design conditions by changing one design value with respect to the standard design condition;
   a property prediction unit configured to predict two or more property values under each of the plurality of neighboring design conditions; and
   a visualization unit configured to arrange symbols corresponding to the plurality of neighboring design conditions, on a plane having the one design value and a first property value among the two or more property values as axes, in a color representation corresponding to a second property value among the two or more property values.
[2] A property prediction device including:
   a standard design determination unit configured to determine a standard design condition representing a design condition in a target substance produced using two or more material substances;
   a neighboring design generation unit configured to generate a plurality of neighboring design conditions by changing two design values with respect to the standard design condition;
   a property prediction unit configured to predict one or more property values under each of the plurality of neighboring design conditions; and
   a visualization unit configured to arrange symbols corresponding to the plurality of neighboring design conditions, on a plane having a first design value and a second design value among the two design values as axes, in a color representation corresponding to the one or more property values.
[3] The property prediction device as described in [2],
   wherein the property prediction unit is configured to predict two or more of said property values, and
   wherein the visualization unit is configured to arrange the symbols at coordinates corresponding to the plurality of neighboring design conditions, regions represented by color representations respectively corresponding to the one or more property values being combined in each of the symbols.
[4] The property prediction device as described in [3], wherein the regions of each of the symbols are formed in a same shape and are arranged in point symmetry.
[5] The property prediction device as described in any one of [1] to [4],
   wherein the standard design determination unit is configured to determine a neighboring design condition corresponding to a symbol selected from the symbols displayed by the visualization unit as the standard design condition, and
   wherein the visualization unit is configured to rearrange, when the standard design condition is newly selected, symbols corresponding to neighboring design conditions generated based on the standard design condition on the plane.
[6] The property prediction device as described in any one of [1] to [4],
   wherein the standard design determination unit is configured to determine the standard design condition by editing a neighboring design condition corresponding to a symbol among the symbols displayed by the visualization unit, and
   wherein the visualization unit is configured to rearrange, when the standard design condition is newly selected, symbols corresponding to neighboring design conditions generated based on the standard design condition on the plane.
[7] The property prediction device as described in any one of [1] to [6],
   wherein the neighboring design generation unit is configured to generate the plurality of neighboring design conditions by changing the design value within a range between a predetermined upper limit value and a predetermined lower limit value and with a predetermined change width.
[8] The property prediction device as described in any one of [1] to [7],
   wherein the property prediction unit is configured to predict the property value by using a machine learning model in which the design value is an explanatory variable and the property value is an objective variable.
[9] A property prediction method including:
   a step of determining, by a computer, a standard design condition representing a design condition in a target substance produced using two or more material substances;
   a step of generating, by the computer, a plurality of neighboring design conditions by changing one design value with respect to the standard design condition;
   a step of predicting, by the computer, two or more property values under each of the plurality of neighboring design conditions; and
   a step of arranging, by the computer, symbols corresponding to the plurality of neighboring design conditions on a plane having the one design value and a first property value among the two or more property values as axes in a color representation corresponding to a second property value among the two or more property values.
[10] A property prediction method including:
   a step of determining, by a computer, a standard design condition representing a design condition in a target substance produced using two or more material substances;
   a step of generating, by the computer, a plurality of neighboring design conditions by changing two design values with respect to the standard design condition;
   a step of predicting, by the computer, one or more property values under each of the plurality of neighboring design conditions; and
   a step of arranging, by the computer, symbols corresponding to the plurality of neighboring design conditions, on a plane having a first design value and a second design value among the two design values as axes, in a color representation corresponding to the one or more property values.
[11] A program for causing a computer to perform a process including:
   a step of determining a standard design condition representing a design condition in a target substance produced using two or more material substances;
   a step of generating a plurality of neighboring design conditions by changing one design value with respect to the standard design condition;
   a step of predicting two or more property values under each of the plurality of neighboring design conditions; and
   a step of arranging symbols corresponding to the plurality of neighboring design conditions, on a plane having the one design value and a first property value among the two or more property values as axes, in a color representation corresponding to a second property value among the two or more property values.
[12] A program for causing a computer to perform a process including:
   a step of determining a standard design condition representing a design condition in a target substance produced using two or more material substances;
   a step of generating a plurality of neighboring design conditions by changing two design values with respect to the standard design condition;
   a step of predicting one or more property values under each of the plurality of neighboring design conditions; and
   a step of arranging symbols corresponding to the plurality of neighboring design conditions, on a plane having a first design value and a second design value among the two design values as axes, in a color representation corresponding to the one or more property values.

### Effect of the invention

According to an aspect of the present disclosure, a relationship between a design value and a property value of a target substance can be easily grasped.

### BRIEF DESCRIPTION OF THE DRAWINGS

[FIG. 1] FIG. 1 is a block diagram illustrating an example of an overall configuration of a design support system.
[FIG. 2] FIG. 2 is a block diagram illustrating an example of a hardware configuration of a computer.
[FIG. 3] FIG. 3 is a block diagram illustrating an example of a functional configuration of the design support system.
[FIG. 4] FIG. 4 is a flowchart illustrating an example of a processing procedure of a property prediction method.
[FIG. 5] FIG. 5 is a diagram illustrating an example of reference design data.
[FIG. 6] FIG. 6 is a diagram illustrating an example of a reference design input screen.
[FIG. 7] FIG. 7 is a flowchart illustrating an example of a procedure of a neighboring design generation process.
[FIG. 8] FIG. 8 is a diagram illustrating a first example of a neighboring design condition.
[FIG. 9] FIG. 9 is a diagram illustrating a second example of the neighboring design condition.
[FIG. 10] FIG. 10 is a diagram illustrating a first example of a property prediction result.
[FIG. 11] FIG. 11 is a diagram illustrating a second example of the property prediction result.
[FIG. 12] FIG. 12 is a diagram illustrating a third example of the property prediction result.
[FIG. 13] FIG. 13 is a diagram illustrating a first example of a property prediction screen.
[FIG. 14] FIG. 14 is a diagram illustrating a second example of the property prediction screen.
[FIG. 15] FIG. 15 is a diagram illustrating a third example of the property prediction screen.
[FIG. 16] FIG. 16 (A) is a diagram illustrating an example of a symbol for displaying two prediction values. FIG. 16 (B) is a diagram illustrating an example of the symbol for displaying two prediction values. FIG. 16 (C) is a diagram illustrating an example of a symbol for displaying three prediction values. FIG. 16 (D) is a diagram illustrating an example of a symbol for displaying four prediction values.
[FIG. 17] FIG. 17 is a diagram illustrating a fourth example of the property prediction result.
[FIG. 18] FIG. 18 is a diagram illustrating an example of a standard design edit screen.

### DESCRIPTION OF EMBODIMENTS

Hereinafter, embodiments of the present invention will be described with reference to the accompanying drawings. Here, in the specification and the drawings, components having substantially the same functional configuration are denoted by the same reference symbols, and duplicated description thereof will be omitted.

### [Embodiments]

Conventionally, material development has been performed based on experience and intuitions of engineers. In recent years, a technique of improving efficiency of material development by utilizing machine learning or the like is used. With respect to the above, when selecting a material or a composition based on a prediction result obtained by machine learning, a final decision is required to be made by a specialized engineer. Therefore, with respect to the prediction result of the machine learning, information indicating a reason why the material or the composition is selected is also required.

To explain a prediction result obtained by a machine learning model, Explainable Artificial Intelligence (XAI) has been proposed. Examples of the XAI technology include Shapley Additive Explanations (SHAP), Local Interpretable Model-agnostic Explainations (LIME), and the like. However, in the related art, it takes time for a user to understand information output by the XAI technology. Additionally, when an uncontrolled explanatory variable is included, it is difficult to utilize the information.

An embodiment of the present disclosure is a design support system configured to support a design of a substance produced using multiple substances. Hereinafter, a substance to be designed is referred to as a "target substance", and a substance used to produce the target substance is referred to as a "material substance".

An example of the target substance in the present embodiment is a resin, an alloy, or the like. Examples of the material substance in the present embodiment include a monomer, a polymer, an additive, or the like. The target substance and the material substance are not limited to these, and any substance can be applied as long as it is a substance produced using multiple substances.

The design support system in the present embodiment generates multiple design conditions (hereinafter, also referred to as "neighboring design conditions") in which one or more design values change in a design condition of the target substance (hereinafter, also referred to as a "standard design condition"), and displays a relationship between the changed one or more design values and one or more property values predicted under each of the neighboring design conditions. The design condition of the target substance includes the amount, the molecular weight, and a physical property (for example, the degree of polymerization of the polymer, the glass transition point, and the like) of each material substance, and a production condition (for example, the processing temperature, the processing time, and the like).

In particular, the design support system according to the present embodiment can display the relationship between the design value and the property value even when multiple design values change or multiple property values are predicted. Therefore, according to the design support system in the present embodiment, the change tendency in the property value due to the change of the design value can be easily grasped.

For example, when predicting one property value for a neighboring design condition obtained by changing two design values of the standard design condition, the design support system according to the present embodiment plots a symbol represented by a color representation corresponding to the property value on a plane having the changed design values as the axes. Therefore, according to the design support system in the present embodiment, even when multiple design values change, the change tendency in the property value can be easily grasped.

Additionally, for example, when predicting two property values for the neighboring design condition obtained by changing one design value of the standard design condition, the design support system according to the present embodiment plots a symbol represented by a color representation corresponding to a second property value on a plane having the changed design value and a first property value as axes. Therefore, according to the design support system in the present embodiment, even when predicting multiple property values, the change tendency in the property values can be easily grasped.

Further, for example, when predicting two property values for the neighboring design condition obtained by changing two design values of the standard design condition, the design support system according to the present embodiment plots a symbol, in which regions represented by color representations respectively corresponding to the property values are combined, on a plane having the changed two design values as axes. Therefore, according to the design support system in the present embodiment, even when predicting multiple property values for the design condition obtained by changing multiple design values, the change tendency in the property values can be easily grasped.

The design support system in the present embodiment can determine a new standard design condition based on the displayed prediction result, and predict one or more property values for a neighboring design condition obtained by changing one or more design values with respect to the new standard design condition. Therefore, according to the design support system in the present embodiment, by using, as the standard design condition, a design condition with which a good prediction result has been obtained, a neighboring design condition, with which a better prediction result can be obtained, can be repeatedly searched for. Therefore, according to the design support system in the present embodiment, the design condition of the target substance can be efficiently determined.

### <Overall Configuration of Design Support System>

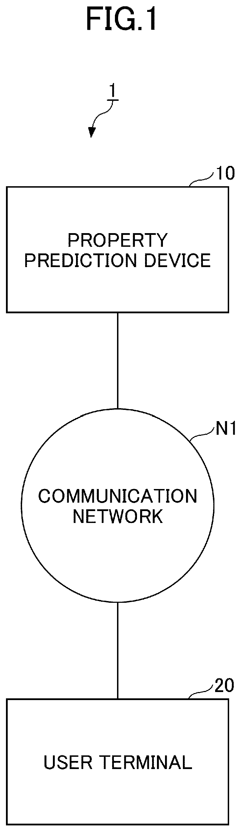

An overall configuration of the design support system according to the present embodiment will be described with reference to FIG. 1. FIG. 1 is a block diagram illustrating an example of the overall configuration of the design support system according to the present embodiment.

As illustrated in FIG. 1, a design support system 1 according to the present embodiment includes a property prediction device 10 and a user terminal 20. The property prediction device 10 and the user terminal 20 are connected to each other via a communication network N1, such as a local area network (LAN) or the Internet so that data communication can be performed.

The property prediction device 10 is an information processing device, such as a personal computer, a workstation, or a server, configured to visualize a relationship between a design value and a property value of the target substance in response to a request from the user terminal 20. The property prediction device 10 receives the basic design condition related to the target substance to be designed from the user terminal 20. The property prediction device 10 predicts a property value under a neighboring design condition generated based on the basic design condition, and transmits information for visualizing the relationship between the design value and the property value of the target substance (hereinafter, also referred to as "visualization information") to the user terminal 20.

The user terminal 20 is an information processing terminal, such as a personal computer, a tablet terminal, or a smartphone, operated by a user. The user terminal 20 transmits the design condition of the target substance to be designed to the property prediction device 10 in response to a user's operation. The user terminal 20 displays the relationship between the design value and the property value of the target substance to the user based on the visualization information received from the property prediction device 10.

Here, the overall configuration of the design support system 1 illustrated in FIG. 1 is an example, and various system configuration examples can be used according to the application and purpose. For example, the property prediction device 10 may be realized by multiple computers or may be realized as a service of cloud computing. Additionally, for example, the design support system 1 may be realized by a stand-alone information processing device having functions to be provided in the property prediction device 10 and the user terminal 20.

<Hardware Configuration of Design Support System>

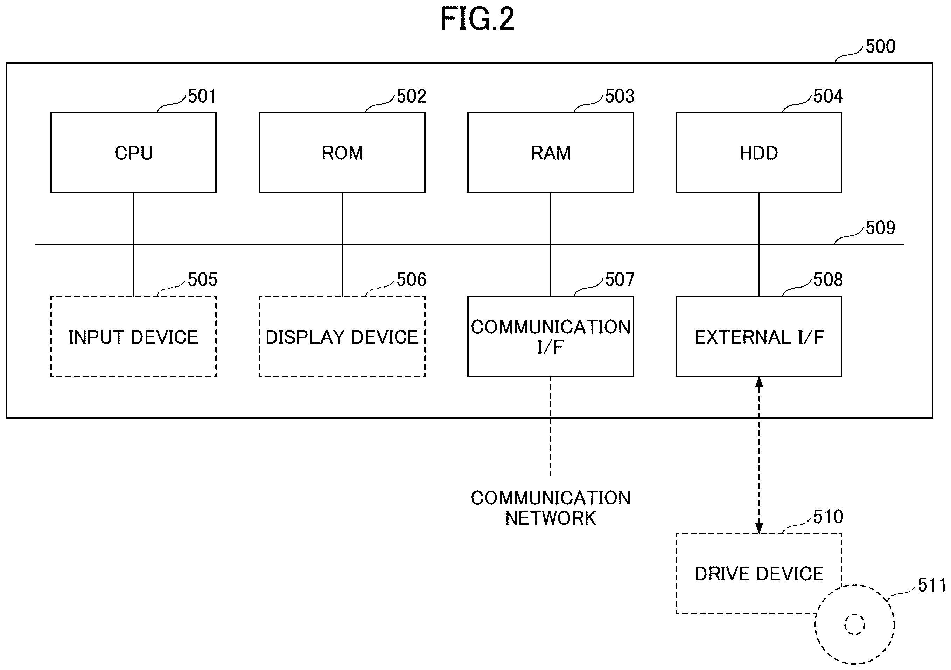

A hardware configuration of the design support system 1 according to the present embodiment will be described with reference to FIG. 2.

### <<Hardware Configuration of Computer>>

The property prediction device 10 and the user terminal 20 in the present embodiment are realized by, for example, a computer. FIG. 2 is a block diagram illustrating an example of a hardware configuration of a computer 500 according to the present embodiment.

As illustrated in FIG. 2, the computer 500 includes a central processing unit (CPU) 501, a read only memory (ROM) 502, a random access memory (RAM) 503, a hard disk drive (HDD) 504, an input device 505, a display device 506, a communication interface (I/F) 507, and an external I/F 508. The CPU 501, the ROM 502, and the RAM 503 form what is called a computer. The hardware components of the computer 500 are connected to each other via a bus line 509. Here, the input device 505 and the display device 506 may be configured to be used by being connected to an external I/F 508.

The CPU 501 is an arithmetic device configured to read a program and data from a storage device, such as the ROM 502 or the HDD 504, onto the RAM 503 and execute processing to realize control and functions of the entire computer 500.

The ROM 502 is an example of a nonvolatile memory (storage device) configured to retain a program and data even when the power is turned off. The ROM 502 functions as a main storage device that stores various programs and data necessary for the CPU 501 to execute various programs installed in the HDD 504. Specifically, the ROM 502 stores a boot program, such as a basic input/output system (BIOS) and an extensible firmware interface (EFI), to be executed at the time of starting the computer 500, operating system (OS) settings, network settings, and the like.

The RAM 503 is an example of a volatile semiconductor memory (storage device) configured to erase a program and information when the power is turned off. The RAM 503 is, for example, a dynamic random access memory (DRAM), a static random access memory (SRAM), or the like. The RAM 503 provides a work area where various programs installed in the HDD 504 are deployed when various programs are executed by the CPU 501.

The HDD 504 is an example of a nonvolatile storage device that stores a program and data. The program and data stored in the HDD 504 include an OS, which is basic software for controlling the entire computer 500, an application for providing various functions on the OS, and the like. Here, the computer 500 may use a storage device (for example, a solid state drive (SSD) or the like) using a flash memory as a storage medium instead of the HDD 504.

The input device 505 is a touch panel, an operation key or button, a keyboard and a mouse, a microphone for inputting sound data, such as voice, or the like, used by a user to input various signals.

The display device 506 is configured by a display, such as a liquid crystal or organic electro-luminescence (EL) display, configured to display a screen, a speaker configured to output sound data, such as voice, and the like.

The communication I/F 507 is an interface that is connected to a communication network and through which the computer 500 performs data transmission.

The external I/F 508 is an interface with an external device. The external device includes a drive device 510 and the like.

The drive device 510 is a device for setting a recording medium 511. The recording medium 511 herein includes a medium for optically, electrically, or magnetically recording information, such as a CD-ROM, a flexible disk, or a magnetooptical disk. Additionally, the recording medium 511 may include a semiconductor memory or the like configured to electrically record information, such as a ROM or a flash memory. With this, the computer 500 can read and/or write data from/to the recording medium 511 via the external I/F 508.

Here, the various programs to be installed in the HDD 504 are installed by, for example, the distributed recording medium 511 being set in the drive device 510 connected to the external I/F 508 and the various programs recorded in the recording medium 511 being read by the drive device 510. Alternatively, the various programs to be installed in the HDD 504 may be installed by being downloaded from another network different from the communication network via the communication I/F 507.

### <Functional Configuration of Design Support System>

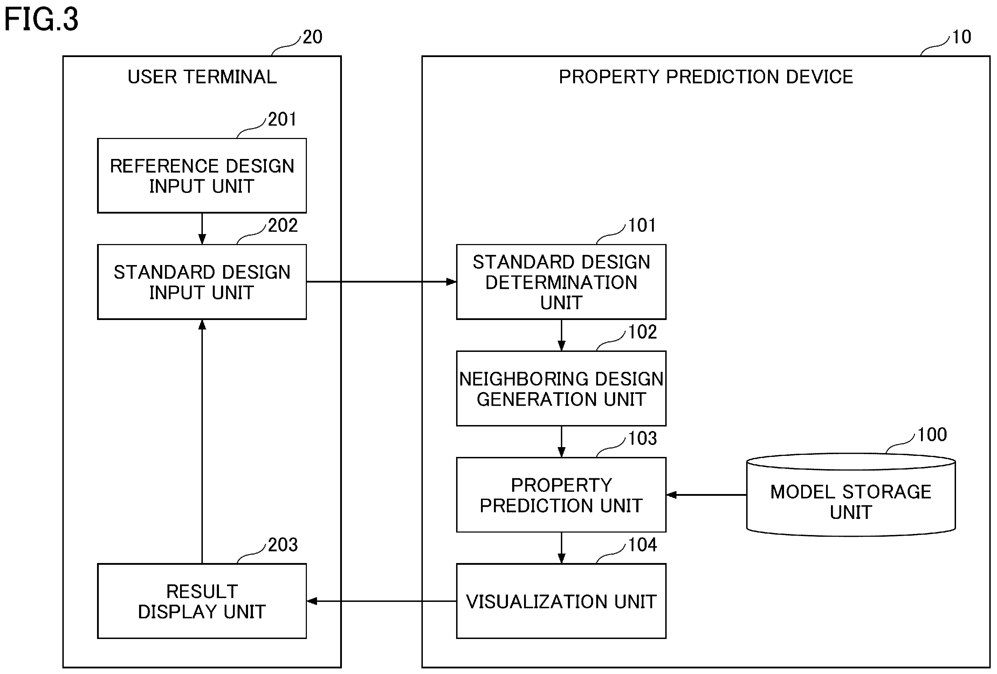

A functional configuration of the design support system according to the present embodiment will be described with reference to FIG. 3. FIG. 3 is a block diagram illustrating an example of the functional configuration of the design support system 1 according to the present embodiment.

### <<Functional Configuration of Property Prediction Device>>

As illustrated in FIG. 3, the property prediction device 10 in the present embodiment includes a model storage unit 100, a standard design determination unit 101, a neighboring design generation unit 102, a property prediction unit 103, and a visualization unit 104.

The standard design determination unit 101, the neighboring design generation unit 102, the property prediction unit 103, and the visualization unit 104 are realized by processing that the CPU 501 is caused to perform by the program deployed from the HDD 504 in the RAM 503 illustrated in FIG. 2. The model storage unit 100 is realized by the HDD 504 illustrated in FIG. 2.

A trained property prediction model is stored in the model storage unit 100. The property prediction model is a machine learning model in which each design value in a design condition of the target substance is set as an explanatory variable and a property value of the target substance is set as an objective variable. An example of the machine learning model is a regression model. The structure of the machine learning model is, for example, a random forest or a deep neural network.

The standard design determination unit 101 receives the design condition selected in response to a user's operation from the user terminal 20. The standard design determination unit 101 determines the received design condition as the standard design condition.

The neighboring design generation unit 102 generates multiple neighboring design conditions in which one or more design values change with respect to the standard design condition. The neighboring design generation unit 102 generates the neighboring design condition by changing the design value of the standard design condition according to a change condition that is set in advance for each design value. The change condition may be suitably set in response to a user's operation.

The property prediction unit 103 predicts one or more property values for each of the neighboring design conditions. The property prediction unit 103 calculates one or more property values by inputting the design values of the neighboring design condition into the trained property prediction model stored in the model storage unit 100.

The visualization unit 104 generates the visualization information for visualizing the relationship between the design value and the property value of the target substance. The visualization unit 104 transmits the generated visualization information to the user terminal 20. The visualization unit 104 visualizes the relationship between the design value and the property value by arranging a symbol represented by a color representation corresponding to the property value, on a plane having the design value or the property value as an axis. In the present embodiment, the design value or the property value represented as the axis of the plane and the property value represented by the color representation can be suitably selected.

### <<Functional Configuration of User Terminal 20>>

As illustrated in FIG. 3, the user terminal 20 in the present embodiment includes a reference design input unit 201, a standard design input unit 202, and a result display unit 203.

The reference design input unit 201, the standard design input unit 202, and the result display unit 203 are realized by processing that the CPU 501 is caused to perform by a program deployed from the HDD 504 in the RAM 503 illustrated in FIG. 2.

The reference design input unit 201 receives an input of reference design data in response to a user's operation. The reference design data is data representing a reference design condition to be a candidate of the standard design condition. The reference design data may include one or more reference design conditions. The reference design input unit 201 displays the received reference design data on the display device 506.

The standard design input unit 202 receives selection of the standard design condition in response to a user's operation. When selecting the standard design condition for the first time, the standard design input unit 202 selects the standard design condition from the reference design data received by the reference design input unit 201. When selecting the standard design condition for the second time or later, the standard design input unit 202 selects the standard design condition from the neighboring design conditions included in the prediction result displayed by the result display unit 203.

The result display unit 203 receives the visualization information from the property prediction device 10. The result display unit 203 displays the relationship between the design value and the property value in the neighboring design condition on the display device 506 based on the received visualization information.

### <Processing Procedure of Design Support System>

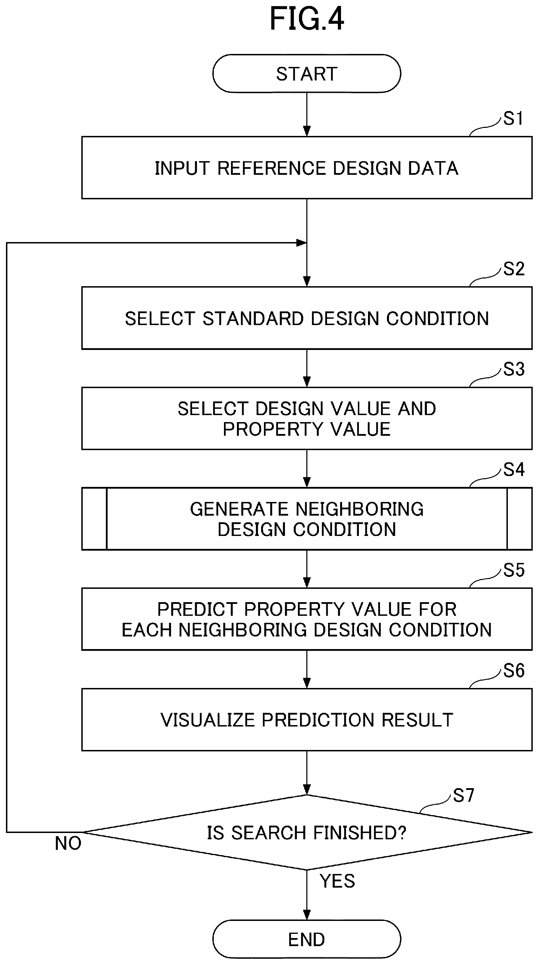

A processing procedure of a property prediction method performed by the design support system 1 in the present embodiment will be described with reference to FIG. 4 to FIG. 17. FIG. 4 is a flowchart illustrating an example of a processing procedure of the property prediction method according to the present embodiment.

In step S1, the reference design input unit 201 included in the user terminal 20 receives an input of the reference design data in response to a user's operation. Next, the reference design input unit 201 displays the received reference design data on the display device 506.

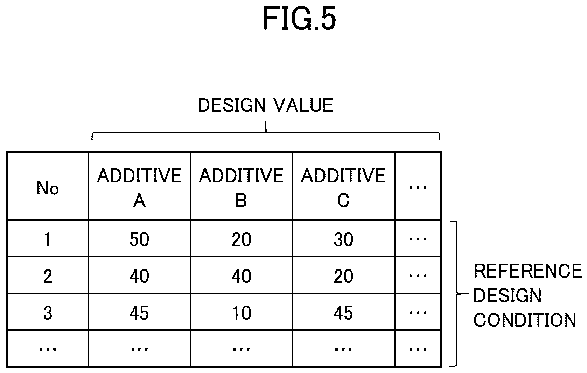

FIG. 5 is a diagram illustrating an example of the reference design data. As illustrated in FIG. 5, the reference design data includes multiple reference design conditions representing design conditions of the target substance. In the example of FIG. 5, the amounts of the material substances used for producing the target substance are used as the design condition.

The input and display of the reference design data are performed on a reference design input screen displayed on the display device 506. The reference design input screen is a screen for receiving the input of the reference design data and displaying the reference design conditions included in the reference design data.

### <<Reference Design Input Screen>>

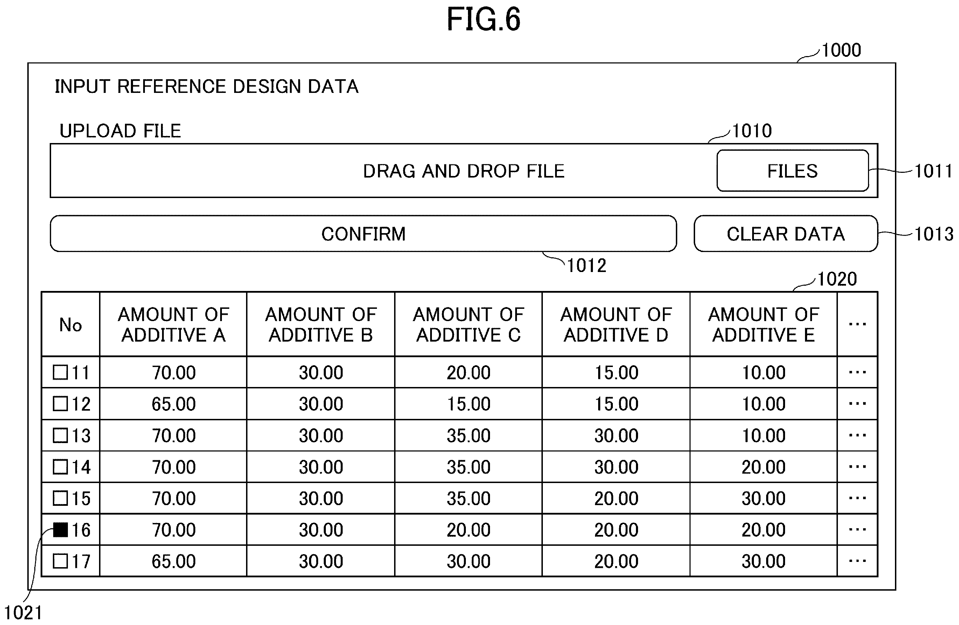

The reference design input screen in the present embodiment will be described with reference to FIG. 6. FIG. 6 is a diagram illustrating an example of the reference design input screen in the present embodiment.

As illustrated in FIG. 6, a reference design input screen 1000 in the present embodiment includes a file input field 1010, a file browse button 1011, a confirmation button 1012, a clear button 1013, and a reference design condition display field 1020.

The user can input the reference design data by dragging and dropping the reference design data in the file input field 1010. The user may input the reference design data by pressing the file browse button 1011 and selecting the reference design data on a displayed file selection screen.

When the reference design data is input by the user, the contents of the reference design data included in the reference design data are displayed in the reference design condition display field 1020 in a selectable manner. In the reference design input screen 1000 illustrated in FIG. 6, the reference design conditions are displayed in a table format in the reference design condition display field 1020, and a selection field 1021 is displayed for each of the reference design conditions.

When the user selects the selection field 1021 corresponding to any one of the reference design conditions and presses the confirmation button 1012, the reference design condition selected in the selection field 1021 is selected as the standard design condition. When the user presses the clear button 1013, the reference design conditions displayed in the reference design condition display field 1020 are erased.

Returning to FIG. 4, the description will be provided. In step S2, the standard design input unit 202 included in the user terminal 20 receives selection of the standard design condition in response to a user's operation. The standard design input unit 202 transmits the selected standard design condition to the property prediction device 10.

In the property prediction device 10, the standard design determination unit 101 receives the standard design condition from the user terminal 20. The standard design determination unit 101 transmits the received standard design condition to the neighboring design generation unit 102.

In step S3, the neighboring design generation unit 102 included in the property prediction device 10 receives the standard design condition from the standard design determination unit 101. Next, the neighboring design generation unit 102 receives selection of one or more design values to be changed and one or more property values to be predicted in response to a user's operation. The selecting of the design value and the property value is performed on the property prediction screen displayed on the display device 506.

In step S4, the neighboring design generation unit 102 included in the property prediction device 10 generates multiple neighboring design conditions obtained by changing one or more design values to be changed with respect to the standard design condition. The neighboring design generation unit 102 transmits the generated multiple neighboring design conditions to the property prediction unit 103.

### <<Neighboring design Generation Process>>

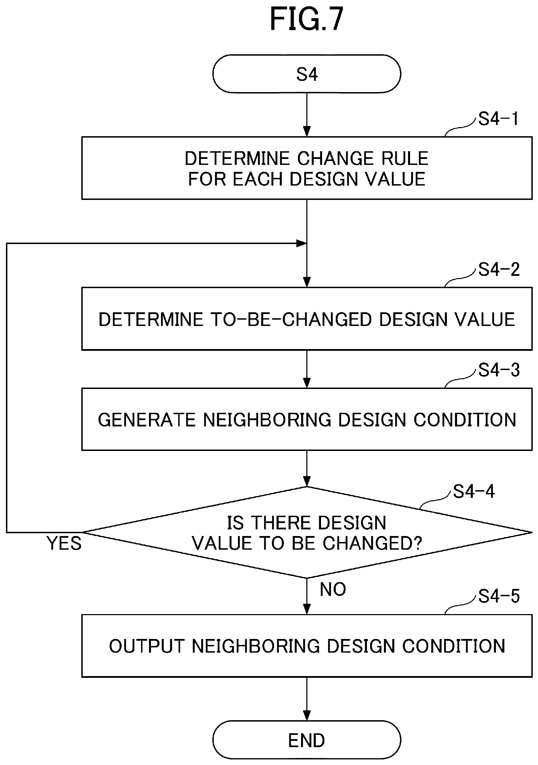

A neighboring design generation process (step S4 in FIG. 4) in the present embodiment will be described in detail with reference to FIG. 7. FIG. 7 is a flowchart illustrating an example of a procedure of the neighboring design generation process according to the present embodiment.

In step S4-1, the neighboring design generation unit 102 determines a change condition for each design value of the design condition. The change condition may be set in advance for each design value, or may be suitably set in response to a user's operation. The change condition in the present embodiment includes an upper limit value, a lower limit value, a change range, and the like. That is, the change condition indicates that the design value to be changed changes in a unit of a predetermined change width in a range of a predetermined lower limit value or greater and a predetermined upper limit value or less.

In step S4-2, the neighboring design generation unit 102 selects a design value to be changed (hereinafter, also referred to as a "to-be-changed design value") from the one or more design values selected to be changed. A criterion for selecting the to-be-changed design value from the design values to be changed is suitably determined. For example, the name of the design value, the order of selection to be changed, and the like may be used.

In step S4-3, the neighboring design generation unit 102 generates the neighboring design conditions by changing the to-be-changed design value with respect to the standard design condition according to the change condition. At this time, the design values other than the to-be-changed design value are fixed. That is, multiple neighboring design conditions in which only the to-be-changed design value is different from that of the standard design condition are generated.

If there is a neighboring design condition that has already been generated, the neighboring design generation unit 102 generates a neighboring design condition by changing the to-be-changed design value according to the change condition for each generated neighboring design condition.

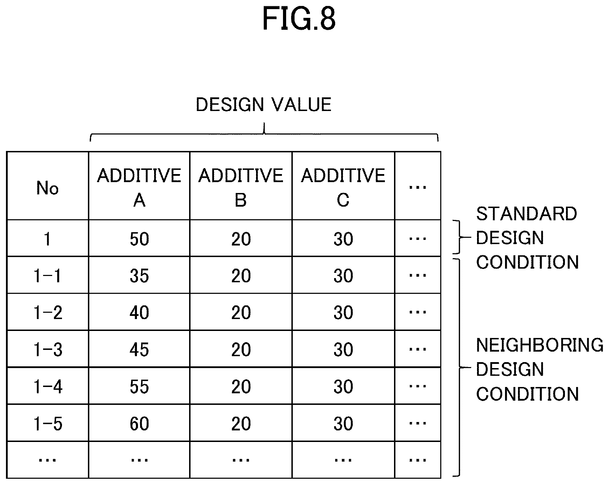

FIG. 8 is a diagram illustrating an example of the neighboring design conditions in which one design value changes with respect to the standard design condition. The example of FIG. 8 is an example in which the reference design condition (No. 1) illustrated in FIG. 5 is selected as the standard design condition, and "amount of additive A", which is an example of the design value, is selected as the to-be-changed design value. As illustrated in FIG. 8, in the multiple neighboring design conditions (No. 1-1 to 1-5), only the amount of the additive A changes with respect to the standard design condition (No. 1), and the other design values are fixed.

Returning to FIG. 7, the description will be provided. In step S4-4, the neighboring design generation unit 102 determines whether there is a design value that is not selected as the to-be-changed design value (in other words, a design value with which the neighboring design condition is not generated) among the design values selected to be changed. If there is a design value that is not selected as the to-be-changed design value (YES), the neighboring design generation unit 102 returns the process to step S4-2. If all the design values have been selected as the to-be-changed design value (NO), the neighboring design generation unit 102 advances the process to step S4-5.

In step S4-2 performed again, the neighboring design generation unit 102 selects a new to-be-changed design value from the design values that are not selected as the to-be-changed design value among the design values selected to be changed. Subsequently, steps S4-3 to S4-4 are performed again for the selected new to-be-changed design value.

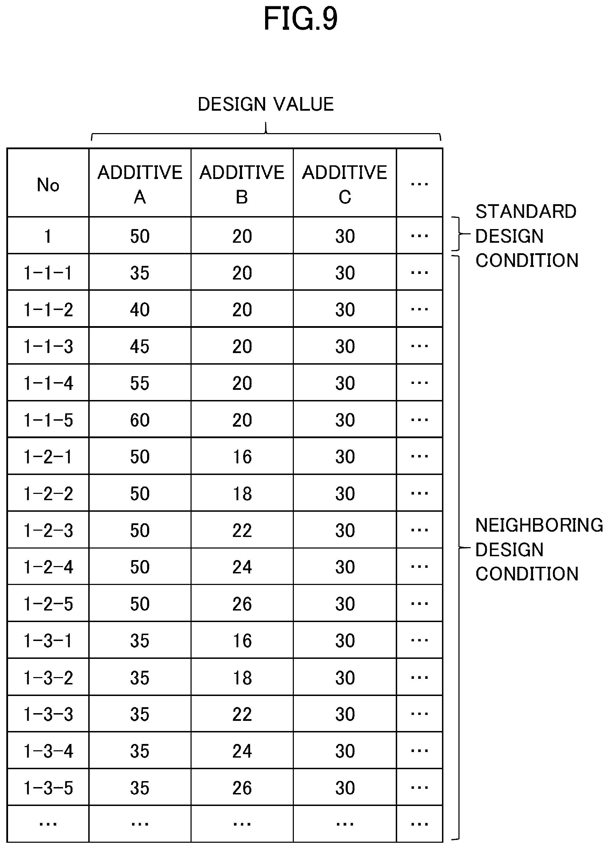

FIG. 9 is a diagram illustrating an example of the neighboring design conditions in which two design values change with respect to the standard design condition. The example of FIG. 9 is an example in which the reference design condition (No. 1) illustrated in FIG. 5 is selected as the standard design condition, and "amount of additive A" and "amount of additive B", which are examples of the design values, are selected as the to-be-changed design values. As illustrated in FIG. 9, multiple neighboring design conditions (No. 1-1-1 to 1-3-5) include neighboring design conditions (No. 1-1-1 to 1-1-5), in which only the amount of the additive A changes, neighboring design conditions (No. 1-2-1 to 1-2-5), in which only the amount of the additive B changes, and neighboring design conditions (No. 1-3-1 to 1-3-5) in which the amount of the additive A and the amount of the additive B change, with respect to the standard design condition (No. 1).

Returning to FIG. 7, the description will be provided. In step S4-5, the neighboring design generation unit 102 transmits the neighboring design conditions generated in step S4-3 to the property prediction unit 103. Here, when step S4-3 is performed multiple times, all the neighboring design conditions generated each time are transmitted to the property prediction unit 103.

Returning to FIG. 4, the description will be provided. In step S5, the property prediction unit 103 included in the property prediction device 10 receives multiple neighboring design conditions from the neighboring design generation unit 102. Next, the property prediction unit 103 reads the trained property prediction model from the model storage unit 100.

Subsequently, the property prediction unit 103 predicts one or more property values to be predicted for each of the neighboring design conditions. The property prediction unit 103 calculates one or more property values by inputting the design values of each of the neighboring design conditions into the trained property prediction model. Then, the property prediction unit 103 transmits the prediction result to the visualization unit 104. The prediction result includes the neighboring design condition and the property value predicted under the neighboring design condition.

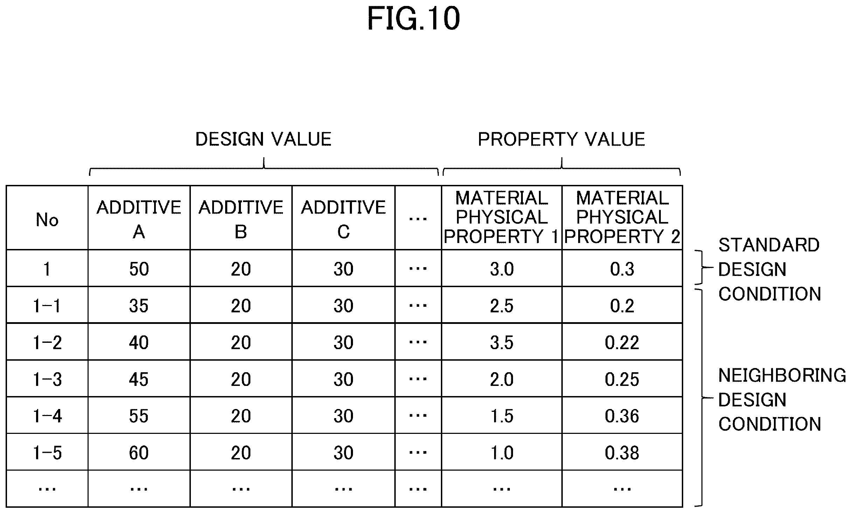

FIG. 10 is a diagram illustrating an example of the prediction result obtained by predicting two property values under each of the neighboring design conditions illustrated in FIG. 8. As illustrated in FIG. 10, "material physical property 1" and "material physical property 2", which are examples of the property value, are predicted for each of the neighboring design conditions (No. 1-1 to 1-5).

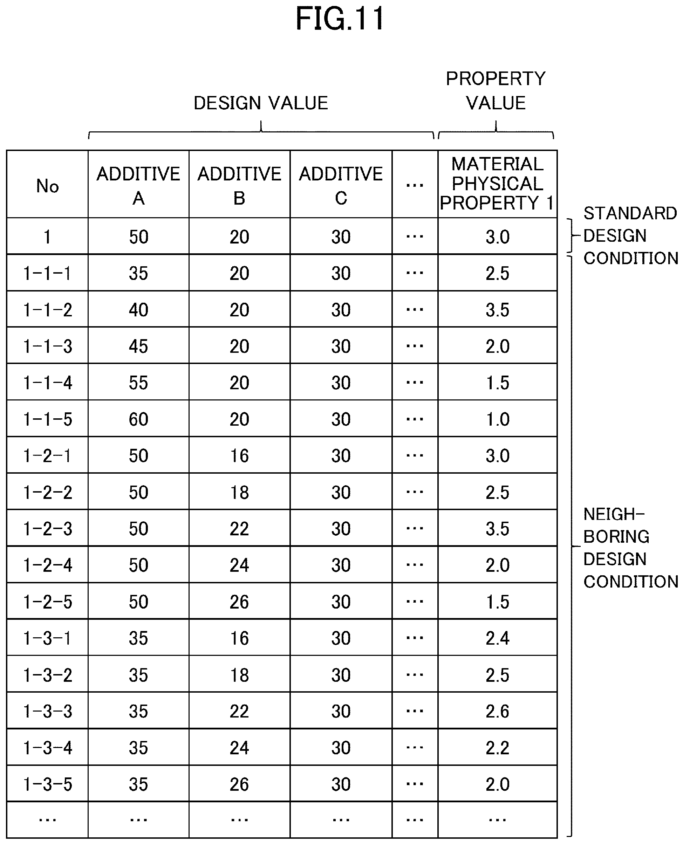

FIG. 11 is a diagram illustrating an example of a prediction result obtained by predicting one property value under each of the neighboring design conditions illustrated in FIG. 9. As illustrated in FIG. 11, "material physical property 1", which is an example of the property value, is predicted for each of the neighboring design conditions (No. 1-1-1 to 1-3-5).

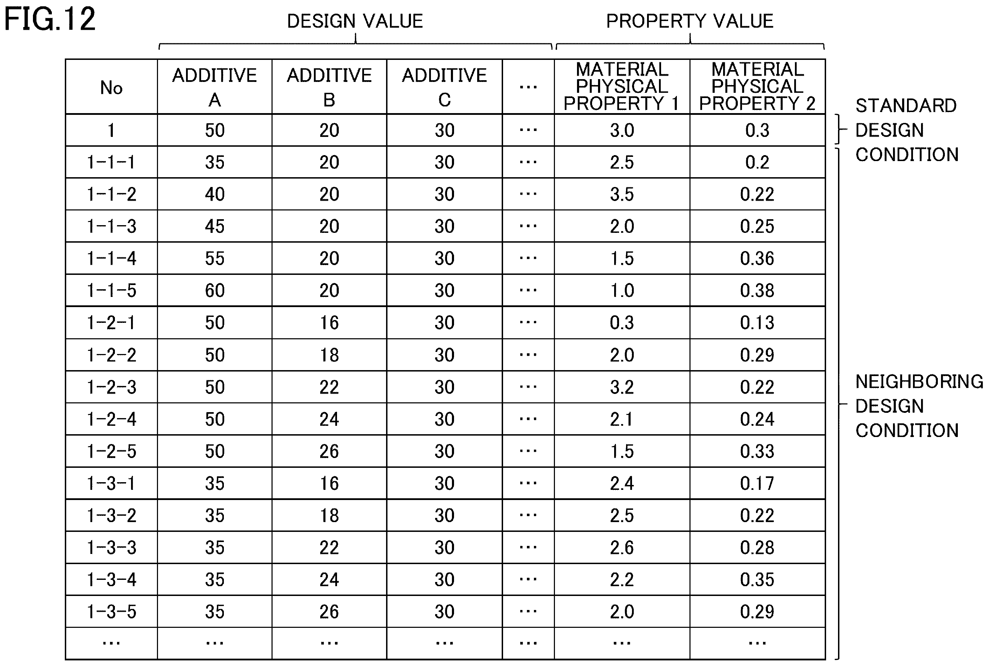

FIG. 12 is a diagram illustrating an example of a prediction result obtained by predicting two property values under each of the neighboring design conditions illustrated in FIG. 9. As illustrated in FIG. 12, "material physical property 1" and "material physical property 2", which are examples of the property value, are predicted for each of the neighboring design conditions (No. 1-1-1 to 1-3-5).

Returning to FIG. 4, the description will be provided. In step S6, the visualization unit 104 included in the property prediction device 10 receives the prediction result from the property prediction unit 103. Next, the visualization unit 104 generates the visualization information for visualizing the relationship between the design value and the property value of the target substance. Subsequently, the visualization unit 104 transmits the visualization information to the user terminal 20.

In the user terminal 20, the result display unit 203 receives the visualization information from the property prediction device 10. The result display unit 203 visualizes the relationship between the design value and the property value of the target substance based on the visualization information. Specifically, the result display unit 203 displays the relationship between the design value and the property value of the target substance on the property prediction screen displayed on the display device 506.

### <<First Example of Property Prediction Screen>>

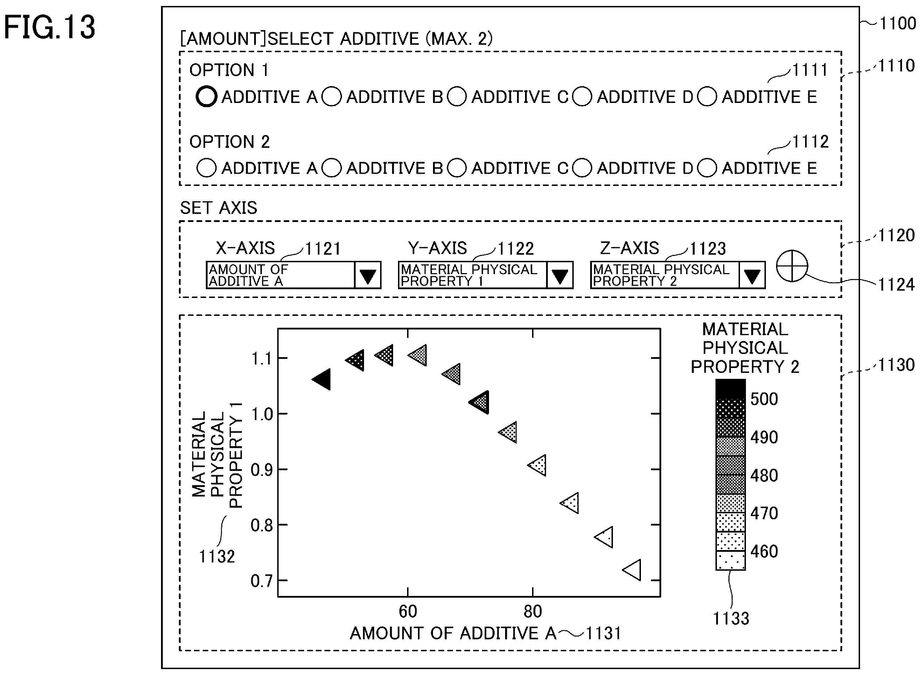

FIG. 13 is a diagram illustrating a first example of the property prediction screen in the present embodiment. The first example of the property prediction screen is a property prediction screen when two property values are predicted under the neighboring design conditions in which one design value changes with respect to the standard design condition.

As illustrated in FIG. 13, a property prediction screen 1100 in the first example includes a design value selection field 1110, an axis setting field 1120, and a graph display field 1130.

The design value selection field 1110 includes a first selection field 1111 and a second selection field 1112. In the first selection field 1111 and the second selection field 1112, design values that can be changed in the design condition are displayed in a selectable manner. In the property prediction screen according to the present embodiment, a list of the design values is displayed in each of the first selection field 1111 and the second selection field 1112 to be exclusively selectable by a radio button. The first selection field 1111 and the second selection field 1112 may be displayed, for example, as a drop-down list or a list box in which to-be-changed design values are options.

The axis setting field 1120 includes an X-axis setting field 1121, a Y-axis setting field 1122, a Z-axis setting field 1123, and an axis addition button 1124. In the X-axis setting field 1121, the design values selected in the design value selection field 1110 are displayed in a selectable manner. In the Y-axis setting field 1122, the design values selected in the design value selection field 1110 or predictable property values are displayed in a selectable manner. The predictable property values are displayed in the Z-axis setting field 1123 in a selectable manner. When the user presses the axis addition button 1124, the Z-axis setting field 1123 is added.

In the property prediction screen according to the present embodiment, in the X-axis setting field 1121, the Y-axis setting field 1122, and the Z-axis setting field 1123, the selected design values or the predictable property values are displayed as a drop-down list in a selectable manner. In the X-axis setting field 1121, the Y-axis setting field 1122, and the Z-axis setting field 1123, for example, a list box or the like having the selected design values or the predictable property values as options may be displayed.

As illustrated in FIG. 13, the first example of the property prediction screen is a display example in the case where two property values are predicted under the neighboring design conditions in which one design value changes. In the example of FIG. 13, the X axis represents the "amount of additive A", which is an example of the design value, the Y axis represents the "material physical property 1", which is an example of a first property value, and the Z-axis represents the "material physical property 2", which is an example of a second property value.

The graph display field 1130 has a plane including an X-axis 1131 and a Y-axis 1132, and a Z-axis 1133 represented by a color bar. In the graph display field 1130, symbols corresponding to the prediction results with respect to the respective neighboring design conditions are arranged at coordinates corresponding to the X-axis 1131 and the Y-axis 1132 in a color representation corresponding to a value of the Z-axis 1133.

The color representation representing the value of the Z-axis may be a color, a gray scale, or a pattern represented by binary values. When the symbols are represented in color, at least one of the hue, the brightness, or the saturation may change in accordance with the property value. For example, two colors separated by 180 degrees in the hue circle may be associated with the minimum value and the maximum value of the property value, and each symbol may be expressed by a hue at an angle corresponding to the predicted property value. When the symbol is represented by a gray scale, the brightness may change in accordance with the property value. For example, the minimum value (0) and the maximum value (255) of the brightness may be associated with the minimum value and the maximum value of the property value, and each symbol may be expressed by the brightness corresponding to the predicted property value.

In the graph display field 1130, the standard design condition and the neighboring design conditions are displayed in a distinguishable manner. In the property prediction screen in the present embodiment, the outer periphery of the symbol corresponding to the standard design condition among the symbols arranged on the XY plane is displayed by a thick line. The method of distinguishing the standard design condition and the neighboring design conditions is not limited thereto, and any method may be used as long as the user can visually recognize the difference of the symbols in color, shape, size, or the like.

In the first example of the property prediction screen, the number of design values to be changed is one, and thus the symbols corresponding to the prediction results in the respective neighboring design conditions are arranged to draw a line graph on the XY plane. The user can grasp a change tendency of the two property values due to the change of the design value by seeing the coordinates and the color expressions of the symbols in a wide view.

### <<Second Example of Property Prediction Screen>>

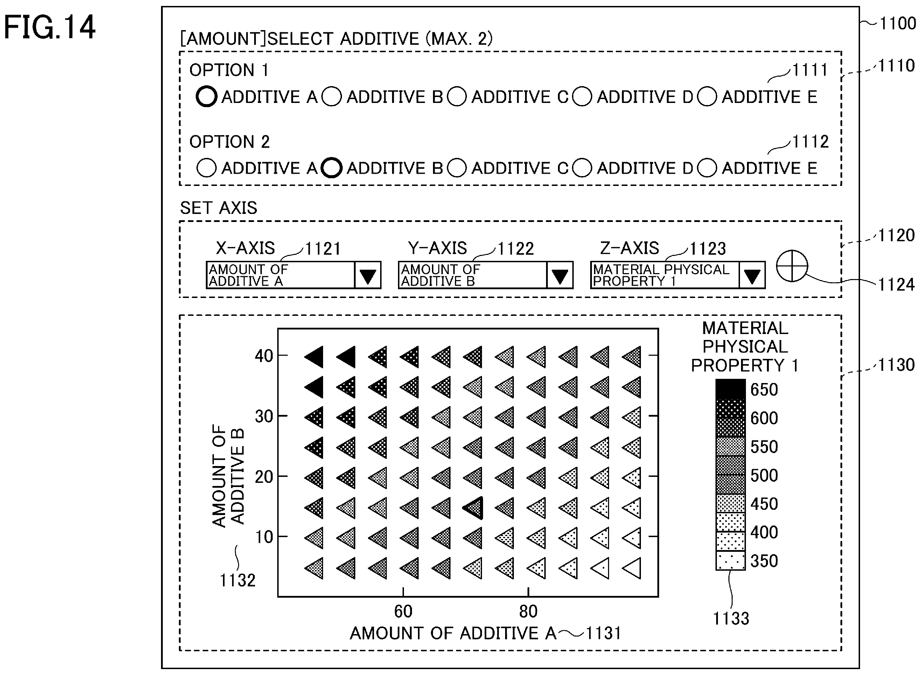

FIG. 14 is a diagram illustrating a second example of the property prediction screen in the present embodiment. The second example of the property prediction screen is a property prediction screen when one property value is predicted under the neighboring design conditions in which two design values change with respect to the standard design condition.

As illustrated in FIG. 14, the second example of the property prediction screen is a display example in the case where one property value is predicted under the neighboring design conditions in which two design values change. In the example of FIG. 14, the X-axis 1131 is "amount of additive A", which is an example of a first design value, the Y-axis 1132 is "amount of additive B", which is an example of a second design value, and the Z-axis 1133 is "material property 1", which is an example of the property value.

In the second example of the property prediction screen, the number of the design values to be changed is two, and thus symbols corresponding to the prediction results under the respective neighboring design conditions are arranged on the entire XY plane. The user can grasp a change tendency of one property value with respect to the combination of two design values by seeing the coordinates and color expressions of the symbols in a wide view.

### <<Third Example of Property Prediction Screen>>

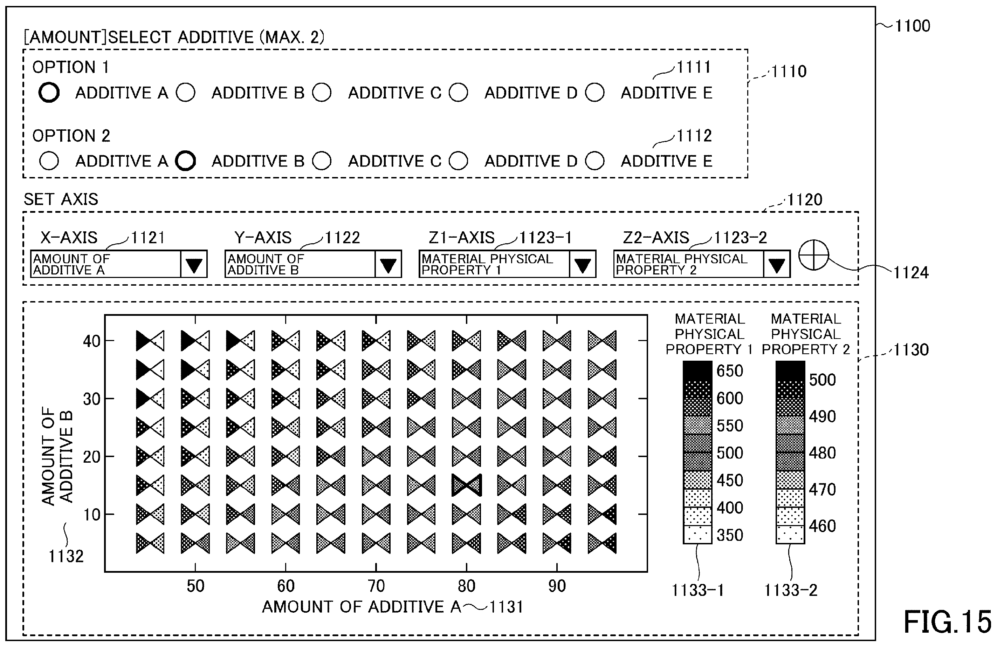

FIG. 15 is a diagram illustrating a third example of the property prediction screen in the present embodiment. The third example of the property prediction screen is a property prediction screen when two property values are predicted in the neighboring design conditions in which two design values change with respect to the standard design condition.

As illustrated in FIG. 15, the third example of the property prediction screen is a display example in the case where two property values are predicted under the neighboring design conditions in which two design values change. In the example of FIG. 15, the X-axis 1131 is "amount of additives A", which is an example of the first design value, the Y-axis 1132 is "amount of additives B", which is an example of the second design value, the Z1-axis 1133-1 is "material property 1", which is an example of the first property value, and the Z2-axis 1133-2 is "material property 2", which is an example of the second property value.

Here, the Z2-axis is displayed by pressing the axis addition button 1124. In this case, the Z-axis in the first example and the second example is the Z1-axis. When three or more property values are predicted, the Z3-axis, the Z4-axis, and ... may be added by further pressing the axis addition button 1124.

In the third example of the property prediction screen, the number of design values to be changed is two, and thus symbols corresponding to the prediction results in the respective neighboring design conditions are arranged on the entire XY plane. Each symbol is formed of a graphic in which a region represented by a color representation corresponding to the Z1-axis and a region represented by a color representation corresponding to the Z2-axis are combined. When three or more property values are predicted, a symbol in which regions represented by color representations respectively corresponding to the property values are combined may be arranged on the XY plane. The user can grasp a change tendency of the two property values with respect to the combination of the two design values by seeing the coordinates of the symbols and the color representations of the regions constituting the symbol in a wide view.

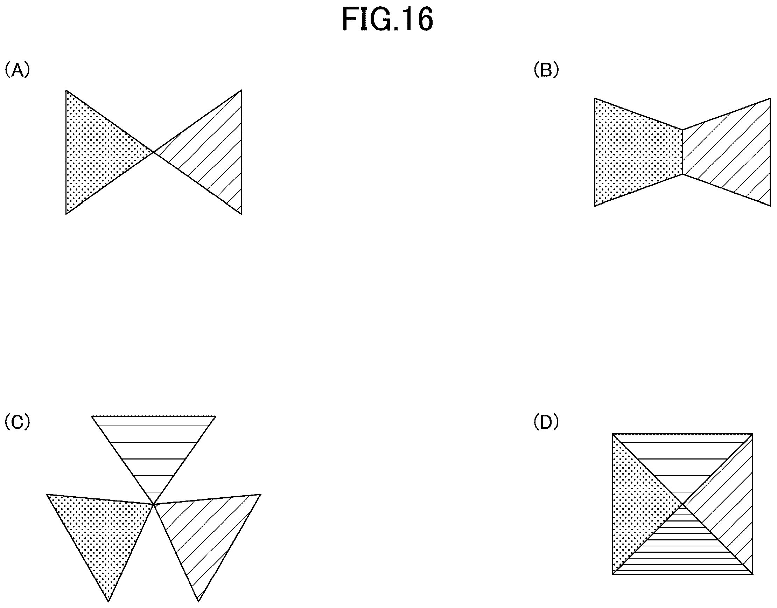

FIG. 16 is a diagram illustrating an example of the symbol in the third example. The symbol in the third example is a symbol in which the regions represented by color representations respectively corresponding to the property values are combined. In the symbol of the third example, it is preferable that the respective regions are formed in the same shape and arranged in point symmetry.

FIG. 16 (A) and (B) are examples of a symbol for displaying two prediction values. In the symbol illustrated in FIG. 16 (A), respective regions are formed in a triangle shape and are arranged to face each other from side to side. In the symbol illustrated in FIG. 16 (B), respective regions are formed in a rectangular shape (trapezoid shape) and are arranged to face each other from side to side.

FIG. 16 (C) is an example of a symbol for displaying three prediction values. In the symbol illustrated in FIG. 16 (C), respective regions are formed in a triangle shape, and are arranged such that the angle formed by the center lines of adjacent triangles is 120°. FIG. 16 (D) is an example of a symbol for displaying four prediction values. In the symbol illustrated in FIG. 16 (D), respective regions are formed in a triangle shape, and are arranged to be combined from side to side and up and down.

The symbol in the third example is not limited to the examples illustrated in FIG. 16. The symbol in the third example is only required to be represented in a form in which the balance of the color and the shape of the region corresponding to each property value can be easily grasped.

Returning to FIG. 4, the description will be provided. In step S7, the visualization unit 104 included in the property prediction device 10 determines whether the search by the user is finished. When the search has been finished (YES), the visualization unit 104 ends the property prediction method. If the search has not been finished (NO), the visualization unit 104 returns the process to step S2.

The determination of whether the search has been finished is performed by determining whether there has been a user operation on the prediction result displayed by the visualization unit 104. When the user performs an operation of selecting a new standard design condition for the prediction result, the visualization unit 104 determines that the search has not been finished. When the user does not perform an operation of selecting a new standard design condition for the prediction result, the visualization unit 104 determines that the search has been finished.

In step S2 performed again, the standard design determination unit 101 determines a new standard design condition in response to a user's operation. Subsequently, the step S3 to step S7 are performed again for the determined new standard design condition.

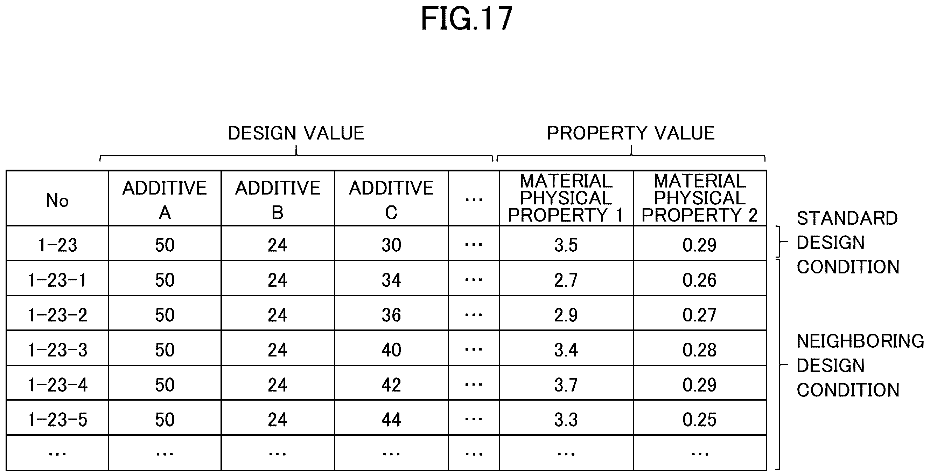

FIG. 17 is a diagram illustrating an example of a prediction result obtained by selecting a new standard design condition from the prediction result and predicting two property values based on the new standard design condition. The example of FIG. 17 is an example in which the neighboring design condition (No. 1-23) is selected as the new standard design condition from the prediction result, and "amount of additive C", which is an example of the design value, is selected as the to-be-changed design value. As illustrated in FIG. 17, in the multiple neighboring design conditions (No. 1-23-1 to 1-23-5), only the amount of the additive C changes with respect to the standard design condition (No. 1-23), and the other design values are fixed. Additionally, "material physical property 1" and "material physical property 2", which are examples of the property value, are predicted for each of the neighboring design conditions (No. 1-23-1 to 1-23-5).

### [Modified Example]

In the design support system 1 according to the embodiment, the configuration in which the new standard design condition is selected from the neighboring design conditions included in the prediction results has been described. In a design support system 1 according to a modified example, a configuration in which a new standard design condition is selected by editing a design value of the neighboring design condition included in the prediction result will be described.

In the design support system 1 according to the present modified example, a design condition edit screen for editing the design value of the neighboring design condition is displayed on the display device 506 of the user terminal 20. The design condition edit screen is displayed by specifying the neighboring design condition to be edited. For example, by selecting a symbol displayed in the graph display field 1130 of the property prediction screen 1100, the design condition edit screen for editing a design value of a neighboring design condition corresponding to the symbol is displayed.

### <<Design Condition Edit Screen>>

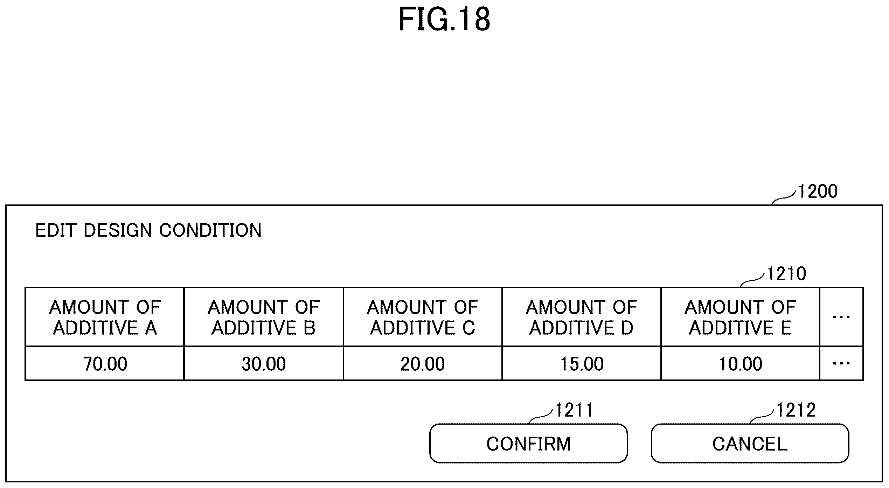

The design condition edit screen in the present modified example will be described with reference to FIG. 18. FIG. 18 is a diagram illustrating an example of the design condition edit screen in the present modified example.

As illustrated in FIG. 18, a design condition edit screen 1200 in the present modified example includes a design condition edit field 1210, a confirmation button 1211, and a cancel button 1212. In the design condition edit field 1210, each design value of the neighboring design condition to be edited is displayed in an editable form. In the design condition edit screen in the present modified example, the design values of the neighboring design condition are displayed in a table format, and each of the design values is displayed to be directly editable.

When the user edits any of the design values in the design condition edit field 1210, the confirmation button 1211 can be pressed. When the user presses the confirmation button 1211, the standard design input unit 202 receives the neighboring design condition edited in the design condition edit field 1210 as the new basic design condition. When the user presses the cancel button 1212, the result display unit 203 closes the design condition edit screen 1200.

The processing after the new standard design condition is selected is substantially the same as that of the design support system 1 in the embodiment described above. That is, the neighboring design conditions in which one or more design values change with respect to the new standard design condition are generated, and the prediction result including each neighboring design condition and one or more property values predicted for each neighboring design condition is displayed on the property prediction screen 1100.

### <Effects of Embodiment>

When predicting one property value with respect to multiple neighboring design conditions obtained by changing two design values of the standard design condition, the design support system according to the present embodiment plots the symbol represented by the color representation corresponding to the prediction value on a plane having the changed design values as axes. Therefore, according to the design support system of the present embodiment, even when multiple design values change, the change tendency in the property value can be easily grasped.

When predicting two property values with respect to multiple neighboring design conditions obtained by changing one design value of the standard design conditions, the design support system in the present embodiment plots, on a plane having the changed design value and one property value as axes, the symbol represented by the color representation corresponding to the other property value. Therefore, according to the design support system of the present embodiment, even when multiple property values are predicted, the change tendency in the property values can be easily grasped.

When predicting two property values with respect to multiple neighboring design conditions obtained by changing two design values of the standard design condition, the design support system according to the present embodiment plots the symbol, in which the regions represented by the color representations respectively corresponding to the property values are combined, on a plane having the changed two design values as axes. Therefore, according to the design support system of the present embodiment, even when multiple property values are predicted with respect to the design conditions in which multiple design values change, the change tendency in the property values can be easily grasped.

The design support system according to the present embodiment can select a desired design condition from the displayed prediction results, and re-display the prediction results obtained by predicting a desired property value with respect to the neighboring design conditions in which the desired design value changes by using the selected design condition as the new standard design condition. Therefore, according to the design support system of the present embodiment, the neighboring design conditions of the design condition for which a good prediction result has been obtained can be repeatedly searched, and the design condition of the target substance can be efficiently determined.

### [Supplement]

Each function of the embodiments described above can be realized by one or more processing circuits. Here, the "processing circuit" in the present specification includes a processor programmed to perform each function by software, such as a processor implemented by an electronic circuit, and a device such as an application specific integrated circuit (ASIC), a digital signal processor (DSP), a field programmable gate array (FPGA), or a conventional circuit module designed to perform each function described above.

Although the embodiments of the present invention have been described in detail, the present invention is not limited to these embodiments, and various modifications or changes can be made within the scope of the gist of the present invention described in the claims.

This application claims priority to Japanese Patent Application No. 2022-119644 filed on July 27, 2022 in the Japan Patent Office, the entire contents of which are incorporated herein by reference.

### Description of reference symbols

1 design support system
10 property prediction device
101 standard design determination unit
102 neighboring design generation unit
103 property prediction unit
104 visualization unit
20 user terminal
201 reference design input unit
202 standard design input unit
203 result display unit

## Claims

1. A property prediction device comprising:
a standard design determination unit configured to determine a standard design condition representing a design condition in a target substance produced using two or more material substances;
a neighboring design generation unit configured to generate a plurality of neighboring design conditions by changing one design value with respect to the standard design condition;
a property prediction unit configured to predict two or more property values under each of the plurality of neighboring design conditions; and
a visualization unit configured to arrange symbols corresponding to the plurality of neighboring design conditions, on a plane having the one design value and a first property value among the two or more property values as axes, in a color representation corresponding to a second property value among the two or more property values.

2. A property prediction device comprising:
a standard design determination unit configured to determine a standard design condition representing a design condition in a target substance produced using two or more material substances;
a neighboring design generation unit configured to generate a plurality of neighboring design conditions by changing two design values with respect to the standard design condition;
a property prediction unit configured to predict one or more property values under each of the plurality of neighboring design conditions; and
a visualization unit configured to arrange symbols corresponding to the plurality of neighboring design conditions, on a plane having a first design value and a second design value among the two design values as axes, in a color representation corresponding to the one or more property values.

3. The property prediction device as claimed in claim 2,
wherein the property prediction unit is configured to predict two or more of said property values, and
wherein the visualization unit is configured to arrange the symbols at coordinates corresponding to the plurality of neighboring design conditions, regions represented by color representations respectively corresponding to the one or more property values being combined in each of the symbols.

4. The property prediction device as claimed in claim 3, wherein the regions of each of the symbols are formed in a same shape and are arranged in point symmetry.

5. The property prediction device as claimed in any one of claims 1 to 4,
wherein the standard design determination unit is configured to determine a neighboring design condition corresponding to a symbol selected from the symbols displayed by the visualization unit as the standard design condition, and
wherein the visualization unit is configured to rearrange, when the standard design condition is newly selected, symbols corresponding to neighboring design conditions generated based on the standard design condition on the plane.

6. The property prediction device as claimed in any one of claims 1 to 4,
wherein the standard design determination unit is configured to determine the standard design condition by editing a neighboring design condition corresponding to a symbol among the symbols displayed by the visualization unit, and
wherein the visualization unit is configured to rearrange, when the standard design condition is newly selected, symbols corresponding to neighboring design conditions generated based on the standard design condition on the plane.

7. The property prediction device as claimed in any one of claims 1 to 6,
wherein the neighboring design generation unit is configured to generate the plurality of neighboring design conditions by changing the design value within a range between a predetermined upper limit value and a predetermined lower limit value and with a predetermined change width.

8. The property prediction device as claimed in any one of claims 1 to 7,
wherein the property prediction unit is configured to predict the property value by using a machine learning model in which the design value is an explanatory variable and the property value is an objective variable.

9. A property prediction method comprising:
a step of determining, by a computer, a standard design condition representing a design condition in a target substance produced using two or more material substances;
a step of generating, by the computer, a plurality of neighboring design conditions by changing one design value with respect to the standard design condition;
a step of predicting, by the computer, two or more property values under each of the plurality of neighboring design conditions; and
a step of arranging, by the computer, symbols corresponding to the plurality of neighboring design conditions on a plane having the one design value and a first property value among the two or more property values as axes in a color representation corresponding to a second property value among the two or more property values.

10. A property prediction method comprising:
a step of determining, by a computer, a standard design condition representing a design condition in a target substance produced using two or more material substances;
a step of generating, by the computer, a plurality of neighboring design conditions by changing two design values with respect to the standard design condition;
a step of predicting, by the computer, one or more property values under each of the plurality of neighboring design conditions; and
a step of arranging, by the computer, symbols corresponding to the plurality of neighboring design conditions, on a plane having a first design value and a second design value among the two design values as axes, in a color representation corresponding to the one or more property values.

11. A program for causing a computer to perform a process comprising:
a step of determining a standard design condition representing a design condition in a target substance produced using two or more material substances;
a step of generating a plurality of neighboring design conditions by changing one design value with respect to the standard design condition;
a step of predicting two or more property values under each of the plurality of neighboring design conditions; and
a step of arranging symbols corresponding to the plurality of neighboring design conditions, on a plane having the one design value and a first property value among the two or more property values as axes, in a color representation corresponding to a second property value among the two or more property values.

12. A program for causing a computer to perform a process comprising:
a step of determining a standard design condition representing a design condition in a target substance produced using two or more material substances;
a step of generating a plurality of neighboring design conditions by changing two design values with respect to the standard design condition;
a step of predicting one or more property values under each of the plurality of neighboring design conditions; and
a step of arranging symbols corresponding to the plurality of neighboring design conditions, on a plane having a first design value and a second design value among the two design values as axes, in a color representation corresponding to the one or more property values.
